# EUROPEAN PATENT SPECIFICATION

(11) **EP 2 046 420 B1**
(45) Date of publication and mention of the grant of the patent: **26.03.2014**
(21) Application number: 07785772.0
(22) Date of filing: 02.08.2007
(51) Int. Cl.: A61M 5/158, A61M 25/06, A61M 5/32

(54) **INSERTION DEVICE**
EINFÜHRVORRICHTUNG
DISPOSITIF D'INSERTION

(30) Priority: 02.08.2006 DK 200601028; 02.08.2006 US 834945 P
(43) Date of publication of application: 15.04.2009
(73) Proprietor: Unomedical A/S, 3460 Birkerød (DK)
(72) Inventor: GYRN, Steffen, 4100 Ringsted (DK); MATHIASEN, Orla, 4180 Sorø (DK)
(74) Representative: Zacco Denmark A/S
(86) International application number: PCT/DK2007/050104
(87) International publication number: WO 2008/014792

(56) References cited:
- WO-A-2005/068006
- WO-A1-2005/046780
- DE-U1- 20 110 059
- US-B1- 6 776 775

## Description

### The technical field

The invention relates to a device for insertion of a cannula of e.g. and infusion set for intermittent or continuous administration of a therapeutical substance, such as e.g. insulin. The insertion device assures that before, during and after insertion the insertion needle is not visible to the patient.

### Background of the invention

The document US patent no. 6.387.078 pertains to an automatic injection apparatus which injects a single, pre-measured dose of stored medicine intramuscularly,or transdermally, and the injection apparatus automatically retracts the hypodermic needle into the device after the injection is completed. The user presses the distal end i.e. the needle end, of the device onto the desired injection site and presses the actuation button. This releases the plunger-syringe-combination from its temporary engagement with the housing. The plunger-syringe-combination together with the spring-to-plunger-coupling are then forced away from the proximal end, i.e. the actuation end, of the housing by an energized driver spring. The driver spring propels the plunger-syringe-combination forward through the bore of the housing until the hypodermic needle exits the housing, and enters the recipient's tissue, and the syringe barrel touches the interior distal end of the housing. During this movement, a return spring positioned between the syringe assembly and the fixed, distal end of the housing becomes compressed and energized. When the liquid of the automatic injection apparatus is discharged by the plunger being pushed forward through the interior of the syringe barrel, the spring-to-plunger-coupling comes into contact with a splitter which disengages the driver spring from the plunger. Without the influence of the driver spring upon the plunger-syringe-combination, the energized return spring forces the plunger-syringe-combination to retreat rearward towards the proximal end of the device until the hypodermic needle is fully retracted into the housing.

As this automatic injection apparatus is directed toward injections of a premeasured dose of stored liquid medicine where the plunger during injection pushes the liquid dose of stored medicine out of the apparatus, the solution will not be applicable for use when inserting an injection device as the handling and injection of a liquid under sterile conditions necessitates a complicated injection apparatus which need to interact with the liquid.

WO 2005/046780 (fig. 97 - 102) describes a device used for automatic insertion of a cannula of an infusion device into the skin of a patient, and afterwards automatic retraction of the insertion needle. The insertion device has the form of an oblong cylinder (length ≈ 4 x diameter) which is open in one end (1984) and provided with means for activation at the other end (1952). When the infusion set has been loaded onto the needle (1968) the lock member (1962) is moved in direction of the end provided with means for activation by the patient using projections (1974) which projections are accessible through a slot (1976) of the housing until barbs (1956) of the lock member (1962) engage an outer surface of the housing (page 26, I. 24-27). Then the open end (1984) is placed against the skin of the patient and the means for activation (1952) is activated. When activated shoulders (1954) on the means for activation engage, the barbs (1956) are pushed toward each other in order to disengage the barbs from the housing. When the barbs are clear of the housing the lock member, the needle hub, the retainer body and the associated infusion device are moved by a first spring in direction of the open end (1984). The inserter device moves the infusion device towards the skin of the patient thereby inserting the needle and the cannula of the infusion device. As the cannula is fully inserted, barbs (1964) of the needle hub (1965) engage ramped surfaces (1972) of the sleeve (1982), causing the barbs (1964) to be forced toward one another. When the barbs (1964) have been forced sufficiently inwardly to clear ends (1988) of the main body (1980), the second spring (1966) then moves the needle hub (1965) in the direction of the activation means (1952). Thus the needle is removed from the infusion device leaving the infusion device in place on the skin while the retainer body remains in a position adjacent the open end of the sleeve so that once the insertion device is removed from the skin of the patient, the retainer body protects the patient from further contact with the needle.

This insertion device is rather complex and used for automatic insertion of a cannula of an infusion device into the skin of a patient. A feature illustrating the complexity of the unit is the fact that the two springs respectively biases the housing from the lock member and the retainer body from the needle hub while a main body is placed between the two spring systems to transfer the force from the first spring to the second spring.

An insertion device for medical devices is also described in DE 201 10 059. The preamble of claim 1 is based on the disclosure of said document. In this document the first insertion part is constituted by the housing 6 and the second insertion part, i.e. the part connected to the injection needle, is constituted by the plunger part 27. The first insertion part according to this device is closed in the distal end where the distal end is the end opposite the end where from the injection needle protrudes during activation. The activation means of this device comprises two buttons 24, 25 on the side of the cylindrical device.

According to the present invention the first insertion part 1 is shaped as a cylindrical tube which tube is open in both ends where the second insertion part 2, which is connected to the injection needle and functions as a plunger, exceeds the distal end of the first insertion part both when the second insertion part is in a retracted and in a forward position relative to the first insertion part. The movable parts of the present invention are relatively large, e.g. the activation means of this device can be constituted by the second insertion part, and as a result the device is very robust and easy to use as the user just by watching the device will be able to predict how it functions.

### Summary of invention

The object of the invention is to provide a simple, non-expensive inserter for an infusion device which inserter would be easy and safe for the user to handle during use and safe to dispose of after use.

The invention concerns a device for insertion of a cannula into a subcutaneous or transcutaneously layer of skin of a patient. The device comprises a first insertion part and a second insertion part, a cannula holding part provided with a cannula, and an injection needle where
- the second insertion part is connected to the injection needle and the injection needle is releasably combined with the cannula of the cannula holding part,
- the first insertion part covers the injection needle in a non-activated position and in an activated position the injection needle projects beyond the first insertion part,
- at least a part of the second insertion part and the first insertion part can be moved in relation to each other between at least one activated position and at least one non-activated position, further the second insertion part exceeds the distal end of the first insertion part.

This position of the second insertion part in relation to the first insertion part makes it possible to use the second insertion part as activation means. In one embodiment the second insertion part is provided with handling means for retraction of the insertion needle from the patients skin.

The cannula can be either a soft cannula which is inserted into the patients skin by a separate insertion needle. The insertion needle is then secured releasably or un-releasably to the second insertion part. The cannula can also be a self-penetrating cannula which stays positioned in the patients skin after insertion. The cannula then is the insertion needle and is the insertion needle/cannula is then only secured to the second insertion part before insertion.

In one embodiment an elastic element supports bringing the second insertion part which is connected to the injection needle from a forward i.e. an activated position to a retracted i.e. a non-activated position. The elastic element can comprise a spring being in contact respectively with a surface of the second insertion part and a surface of the first insertion part.

According to one embodiment at least a part of the second insertion part moves between at least one forward position and at least one retracted position surrounding the first insertion part.

According to one embodiment the second insertion part and the first insertion part are provided with interacting guiding means for guiding the a slidable movement over a certain distance of the first and second insertion parts in relation to each other.

According to one embodiment of the device the injection needle is unreleasably connected to the second insertion part. This is the case if the insertion device is intended for single-use.

According to a second embodiment the injection needle is releasably connected to the second insertion part. This is the case if the insertion device or at least a part of the insertion device is intended for multiple-use. If the insertion device is intended for multiple-use the first and the second insertion parts can be releasably connected to each other and then one part is reusable while the other part is disposable.

According to one embodiment the first insertion part comprises means for releasably connecting to a corresponding receiving portion placed on a patient's skin e.g. the receiving portion is part of a base plate fastened to the patients skin.

According to one embodiment of the invention the proximal end of the first insertion part is shaped as a cylindrical tube which can be releasably connected to a corresponding cylindrical portion placed on a surface on the skin of a patient, e.g. the receiving portion is attached to a base part which is positioned on a patient's skin before injection of the cannula-holding part.

According to one embodiment an elastic element supports bringing the second infusion part from a forward to a retracted position, the elastic element can e.g. be in the form of a helix metal spring, a rubber element or the like.

According to one embodiment the elastic element is unbiased or only slightly biased when the first and the second insertion parts are in a non-activated position.

According to one embodiment the first insertion part is supported against a surface while the guiding means guide the injection needle of the second insertion part through the surface and back again.

According to one embodiment the first insertion part is formed as a pipe section or a tube piece while the second insertion part is at least partly formed as a plunger corresponding to the interior of the first insertion part i.e. a part of the second insertion part can slide between a forward and a retracted position guided by the interior walls of the first insertion part. According to one embodiment the insertion device is provided with means for manually injecting the injection needle (6) by manually pushing the second insertion part towards the surface of injection. Further retraction of the injection needle can also be done by manually pulling the second insertion part away from the surface of injection.

According to one embodiment the needle holding part of the second insertion part including the injection needle is retained in the first insertion part when the first and the second insertion parts are released from each other.

The first insertion part is shaped as a tube piece being open in both ends and having a round or angular transverse profile.

The insertion device can comprise locking means which locking means can lock the position of the first insertion part in relation to the second insertion part in the non-activated position. The locking means can comprise a protruding part positioned on the outer surface of the first insertion part combined with an opening or slit in the surface of the second insertion part.

### Definitions

Distal - in the present text the word distal refers to parts which are far way from the patient's skin in the position the device takes during insertion, normally as far away as possible.
Proximal - in the present text the word proximal refers to parts which are close to the patient's skin in the position the device takes during insertion, normally as close as possible.

### Short description of the drawings

Embodiments of the invention will now be described with reference to the figures in which:
Figure 1 is a cross-sectional view of a first embodiment of an insertion device of the invention where the insertion device is mounted in a receiver and the insertion needle is in a retracted position before activation;
Figure 2 is a cross-sectional view of the embodiment of figure 1 in a position after activation of the insertion device and insertion of the cannula;
Figure 3 is a side view of an insertion device of the embodiment shown in figure 1 before mounting in a receiver;
Figure 4A is a side view of an insertion device of the embodiment shown in figure 1 after mounting in a receiver before activation, figure 4B-F are side views of base parts provided with a peripheral or central rectangular receivers for a cannula holding part with rectangular or round profile;
Figure 5 is a cross-sectional view of another embodiment of an insertion device of the invention where the insertion device is mounted in a receiver and the insertion needle is in a retracted position before activation;
Figure 6 is a cross-sectional view of the embodiment of figure 5 in a position after activation of the insertion device and insertion of the cannula;
Figure 7 is a side view of an insertion device of the embodiment in figure 5 where the first insertion part and the second insertion part are assembled;
Figure 8 is a side view of an insertion device of the embodiment in figure 5 where the first insertion part is disassembled from the second insertion part;
Figure 9 is a side view of the first insertion part of an insertion device of the embodiment in figure 5;
Figure 10 is another side view of the first insertion part of an insertion device of the embodiment in figure 5;
Figure 11 is a side view of the insertion device of the embodiment in figure 5 where the first insertion part is placed in the second insertion part;
Figure 12 is a side view of the insertion device of the embodiment in figure 11 where the first insertion part is rotated to the right;
Figure 13 is a side view of the insertion device of the embodiment in figure 11 where the insertion device is in a position ready for activation and insertion of a cannula;
Figure 14 is a side view of the insertion device of the embodiment in figure 11 where the insertion device is mounted in a receiver ready for insertion;
Figure 15 is a side view of the insertion device of the embodiment in figure 11 where the insertion device is activated and insertion of a cannula is performed;
Figure 16 is a side view of the insertion device of the embodiment in figure 11 where the insertion device is in a position after activation and insertion of a cannula and where the insertion needle is in a retracted position;
Figure 17 is a side view of the first insertion part of the insertion device of the embodiment in figure 11 where the first insertion part is removed from the second insertion part for disposal.

### Detailed description of the preferred embodiments

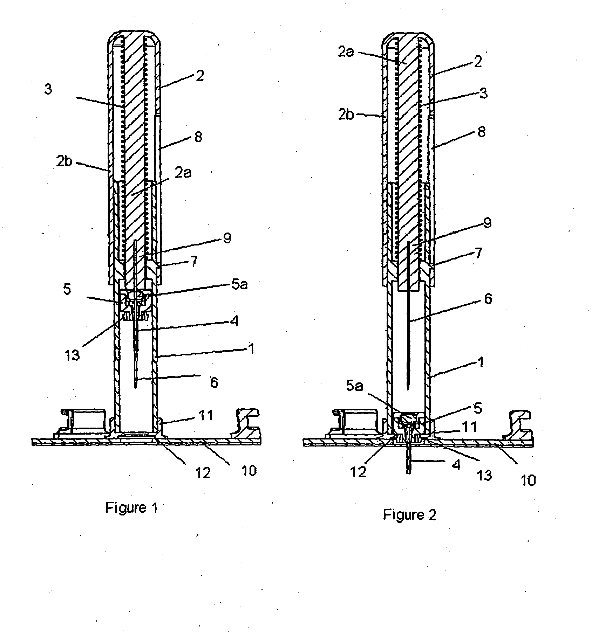

Figures 1 and 2 show a first embodiment of an insertion device according to the invention in a non-activated position. Figure 1 shows the insertion device 1, 2 in a not yet activated position and figure 2 shows the insertion device 1, 2 in a position after having been activated and returned to the non-activated position.

The insertion device 1, 2 shown in figure 1 and 2 comprises a first insertion part 1 and a second insertion part 2, which first insertion part 1 is formed like a cylindrical tube-piece being open in both ends and mounted slidably within the second insertion part 2, the first insertion part 1 comprises guiding means in the form of a tap 7 moving in a slit 8 of the second insertion part 2. The insertion device 1, 2 further comprises an elastic element 3 for returning the first insertion part 1 to the non-activated position after activation, in the non-activated position the elastic element 3 is un-biased or only slightly biased. The insertion device 1, 2 also comprises a needle-holding part 9 with an insertion needle 6 for inserting a cannula-holding part 5 which cannula-holding part 5 comprises a cannula 4 and a septum 5a, into the skin of a patient. The insertion device 1, 2 can be releasably connected to a receiver 11 in which receiver 11 the cannula-holding part 5 can be positioned and secured via hooks 12 on the receiver 11 engaging with recesses 13 formed in the cannula-holding part 5. The receiver 11 is mounted on a base part 10 which base part 10 can be fastened to the patient's skin e.g. with an adhesive mounting pad.

The embodiment of fig. 1 and 2 is intended for single-use of the whole of the insertion device 1, 2. The embodiment makes it possible to first position the receiver 11 via a base plate 10 on the skin of the patient, thereafter to insert the cannula-holding part 5 of the insertion device 1, 2 and to throw the insertion device 1, 2 with the used insertion needle 6 away after insertion of the cannula-holding part 5 which can e.g. have the form of an infusion set. The insertion needle 6 is during injection never visible to the user and the surroundings are protected from the pointy needle.

According to the embodiment of fig 1 and 2 the receiver 11 on the base plate 10 is shaped as an upright positioned cylindrical protrusion, the receiver 11 being for firmly positioning of the first insertion part 1 thereto as well as for receiving and fastening of the cannula-holding part 5. The receiver 11 is fastened unreleasably to the base part 10 and can e.g. be either moulded together with the base plate 10 or fastened to the base plate 10 after the base plate 10 has been formed e.g. by gluing or welding. According to figure 1 and 2 the base part 10 is illustrated as a relatively flat part but base part 10 could be any construction which makes it possible to unite the receiver 11 and the base part 10 into one unit which preferably can be worn by the user directly on the skin. The receiver 11 can be positioned on a peripheral part or a central part of the base plate 10, to accommodate different injection angles for the cannula-holding part 5. The cannula-holding part 5 can be inserted at an angle A deviating from 90° in relation to the distal surface of the base plate 10, normally the angle A will be between 110° and 170° where the distal surface of the base plate 10 form one side of the angle and the inserted cannula 4 form the other side of the angle. In the embodiment of fig. 1 and 2 the receiver 11 is positioned centrally on the base plate 10 with an insertion angle A at 90°.

As shown in figure 1 and 2 the receiver 11 is provided with fastening means comprising hooks 12 placed perpendicular to the upright cylindrical protrusion and parallel to the base plate 10. The hooks 12 correspond to grooves or recesses 13 formed in the cannula-holding part 5. The interaction between the recesses 13 of the cannula-holding part 5 and the hooks 12 of the receiver 11 on the base part 10 assures fastening of the cannula-holding part 5 to the receiver 11 after activation of the insertion device. When the cannula-holding part 5 and the receiver 11 after insertion are connected and fastened to each other they constitute e.g. a gateway for injection or an infusion part which can be joined to a connector part or a positioning part for e.g. of a delivery part comprising a reservoir and a pump.

As shown in figure 1 and 2 the insertion device 1, 2 is a two-part unit, where each unit can e.g. be constructed of a moulded body. The first insertion part 1 and the second insertion part 2 are both formed as cylindrical tubes, but other forms or profiles for the tube such as hexagonal, octagonal or the like can be used. On delivery this single-use equipment can either be jointed to the receiver 11 when delivered to the user in a sterile packing or it can be packed alone. If the single-use equipment is joined to the receiver 11 in a sterile packing, then the user will first unpack the joined device and prepare the base part 10 for fastening to the patients skin e.g. by removing a release liner from a mounting pad, then the user will secure the base plate 10 to the patients skin and finally the user will inject the cannula-holding part 5 and remove the insertion device 1, 2 and dispose of this. If the single-use equipment is packed alone, the user will first unpack the insertion device 1, 2 from the sterile packing, then place the insertion device against the patients skin e.g. in a receiver of a previously positioned base plate 10 and then finally the user will inject the cannula-holding part 5 and remove the insertion device 1, 2 and dispose of the insertion device 1, 2 which now has a contaminated insertion needle 6.

The first insertion part 1, which is positioned in the receiver 11 on the base plate 10, and the second insertion part 2 engage with each other. The second insertion part 2 comprises the needle-holding part 9 carrying the insertion needle 6 for penetrating the skin of a patient and in this present single-use embodiment the second insertion part 2 actually constitutes the needle-holding part 9. The cannula-holding part 5 which is carrying the soft cannula 4 is somehow secured to the needle-holding part 9, normally just by friction between the insertion needle 6 and the soft cannula 4. The insertion device 1, 2 is further provided with an elastic element 3 which holds the two insertion parts 1 and 2 in position i.e. the elastic element 3 forces the first and the second insertion parts 1, 2 into the non-activated position. In this embodiment the elastic element 3 is constituted of a helix metal spring, but the elastic element 3 may take any form, e.g. a rubber cylinder or the like that can force the first and second insertion parts 1, 2 into the non-activated position. When the insertion device 1, 2 is in the position before activation as shown in figure 1, the elastic element 3 is unbiased and when the insertion device 1, 2 is activated by manually pressing down the second insertion part 2 for insertion of the cannula-holding part 5, then the elastic element 3 is biased.

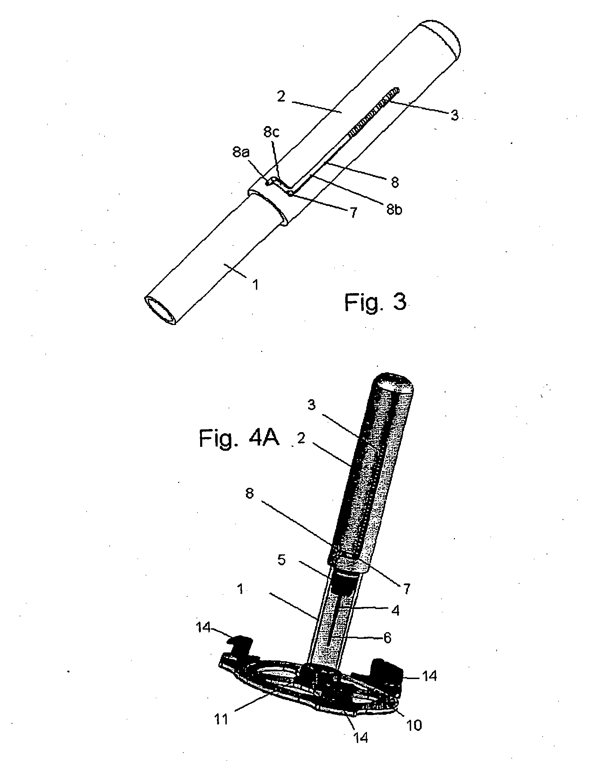

As shown in detail in figure 3, the exterior surface of the first insertion part 1 is provided with a protrusion formed as a cylindrical tap 7 positioned at a part of the exterior surface of the first insertion part 1 which is facing towards an inner surface of the second insertion part 2. The tap 7 interacts with an opening in the form of a slit 8 in the second insertion part 2, said slit 8 comprising three parts 8a, 8b and 8c shaped essentially as an Z with a long part 8b being almost parallel to the insertion direction, a short part 8c being essentially perpendicular to the insertion direction and a short part 8a being angled compared to the short part 8c. When the tap 7 of the first insertion part 1 is placed in a locking position in the slit 8, i.e. the tap is positioned at one of the short part 8a and 8c of the slit 8, the insertion device is then locked in the insertion direction. If the elastic element 3 is slightly biased in the non-activated position then the tap 7 of the first insertion part 1 is forced into the most proximal end of the short part 8a of the slit 8. The first insertion part 1 of this embodiment provides a needle protector both before and after activation of the insertion device 1, 2.

Figure 4A shows the same single-use insertion device as shown in fig. 1 and 2 at an angle from above where the insertion device 1, 2 is ready for activation, i.e. same position as in figure 1. The flexible base plate 10 is provided with fastening means 14 for fastening of a delivery device (not shown) which delivery device can comprise e.g. both a reservoir for medication and a transporting means in the form of a pump or the like.

When activating the single-use insertion device 1, 2 shown in figure 1, 2 and 4, with the intend of fastening of a cannula-holding part 5 in the form of e.g. an infusion set, an injection part or a gateway 5,11 to the skin of the patient, the second insertion part 2 is first rotated around the longitudinal axis defined by the insertion direction in order to move the tap 7 from the short parts 8a and 8c of the slit 8 into the corner of the short part 8c and the long part 8b of the slit 8 thereby making it possible for the tap 7 to move in the longitudinal direction of the long part 8b of the slit 8. The second insertion part 2 is then manually pressed down towards the patient thereby biasing the spring 3 and at the same time the tap 7 move in the long part 8b of the slit 8 towards the distal end of the second insertion part 2, whereby the needle-holding part 9 and the cannula-holding part 5 are being moved, due to the pressing of the second insertion part 2, simultaneously towards the skin of a patient. The insertion needle 6 penetrates the skin and the cannula is inserted into the patient when the insertion device, enters into the fully activated position. As shown in figure 2 the hooks 12 of the receiver 11 engage with the recesses 13 of the cannula-holding part 5 thereby fastening the cannula-holding part 5 to the receiver 11. When the cannula-holding part 5 is fastened to the receiver 11, the pressure on second insertion part 2 is released by removing the manually applied pressure, leading to the spring 3 moving into an unbiased position thereby moving the tap 7 in the slit 8 toward the most proximal end of the slit 8 and at the same time moving the needle 6 into the interior of the first insertion part 1. The second insertion part 2 is then rotated and the tap 7 moved into the short part 8c of the slit 8 for locking the first 1 and second insertion part 2 and at the same time to keep the used needle 6 in place inside the insertion device 1,2. This embodiment provides a single-use insertion device 1, 2 with needle protection, where the needle 6 is never visible to the user and which insertion device 1, 2 can be thrown-away after use without the risk of injuries caused by a pointy needle.

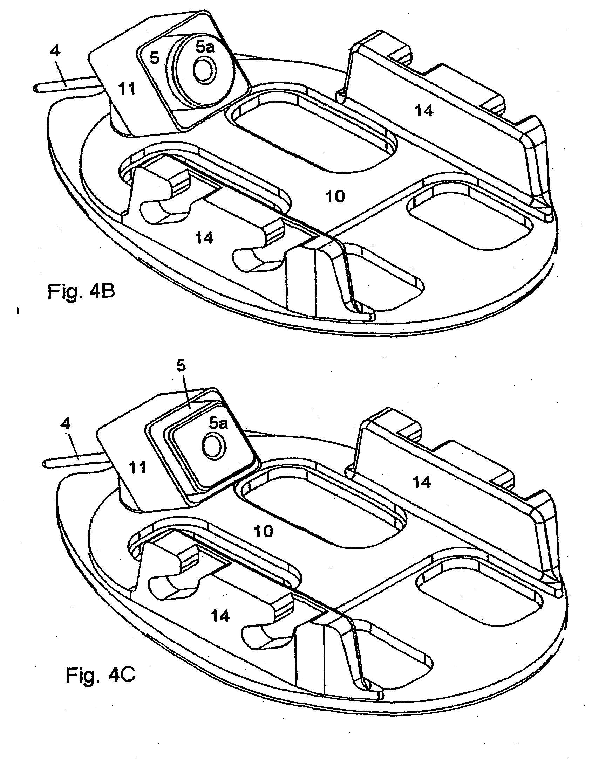

Fig. 4B-F show other embodiments of the base part 10 and a corresponding cannula-holding part 5 correctly positioned by an insertion device 1, 2 according to the invention.

In fig. 4B the peripheral placed receiver 11 as a square profile in which a cannula-holding part 5 having a round profile is placed. In order to position this cannula holding part 5 correctly in the base part 10 an insertion device 1, 2 having a square outer and a round inner profile is needed or at least an inserter which have parts or surfaces adapted to fit into an outer square space formed by the receiver and have parts or surfaces which can provide a space in which a round cannula holding device can slide.

In fig. 4C the peripheral placed receiver has a square profile in which a cannula-holding part 5 having a square profile is placed. In order to position this cannula holding part 5 correctly in the base part 10 an insertion device 1, 2 having a square outer and a square inner profile is needed or at least an inserter which have parts or surfaces adapted to fit into an outer square space formed by the receiver and have parts or surfaces which can provide a space in which a square cannula holding device can slide.

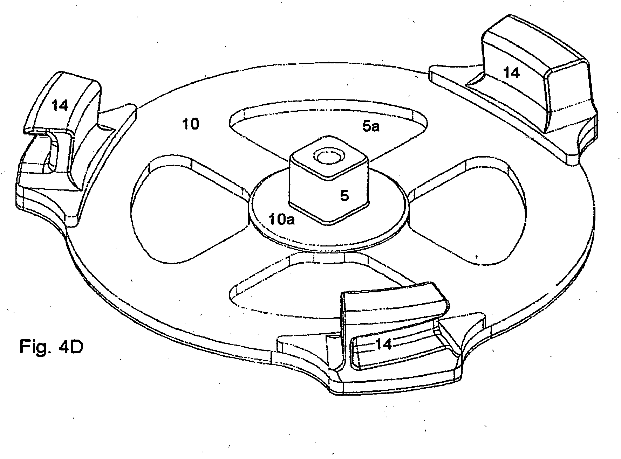

Fig. 4D shows a centrally placed receiver without upright walls guiding the inserter into position. Instead the slightly raised circumference of the central plate 10a of the base part 10 corresponding to a part of the proximal end of the inserter indicates the correct position of the inserter during insertion of the cannula-holding part 5.

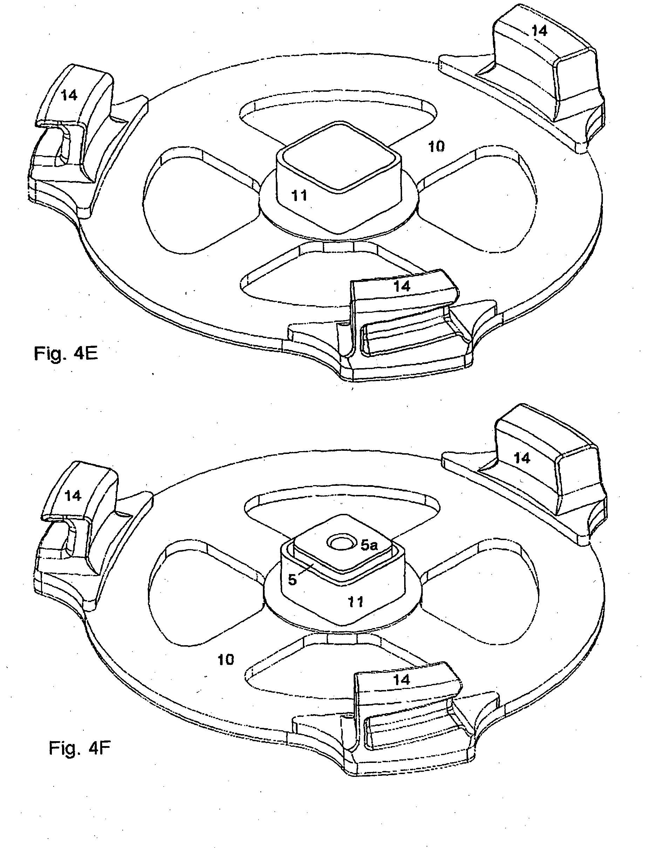

Fig. 4E shows a base part 10 having a centrally placed receiver having upright walls which walls provide the receiver with a square profile. The base part is shown before the cannula-holding part 5 inserted.

Fig. 4F shows a centrally placed receiver 11 having a square profile in which a cannula-holding part 5 having a square profile is placed.

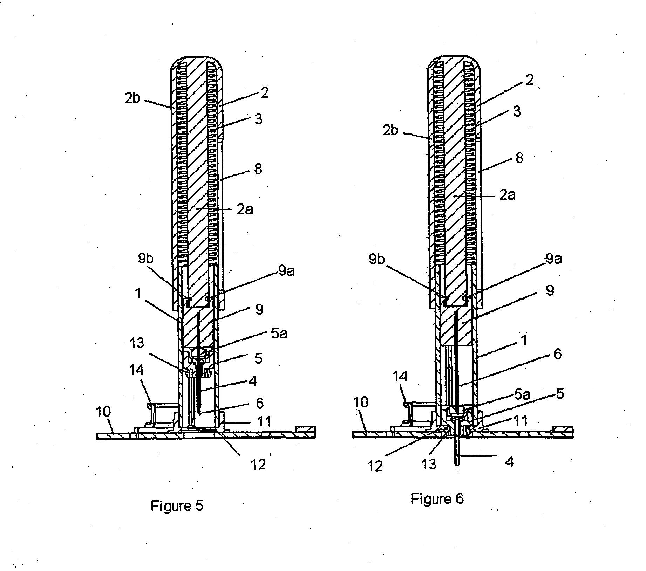

In another embodiment as shown in figure 5 and 6 the insertion device 1, 2 is intended for multiple-use. Figure 5 shows the insertion device 1,2 in a not yet activated position before insertion and figure 6 shows the insertion device 1,2 in a position after activation, where the cannula is inserted.

As shown in figure 5 and 6 the insertion device 1,2 comprises a first insertion part 1 and a second insertion part 2, which first insertion part 1 is mounted slidably within the second insertion part 2 with an elastic element 3 for keeping the first insertion part 1 and the second insertion part 2 in position and for activating the insertion device 1,2, a cannula-holding part 5 provided with a cannula 4 and a septum 5a, the cannula-holding part 5 being connected to a needle-holding part 9, which needle-holding part 9 is provided with an insertion needle 6 for insertion of the cannula 4 into the skin of a patient. In this embodiment the needle-holding part 9 is a separate needle-holding part 9 being releasably fastened to the insertion part 2 by way of example a tongue and groove connection 9a, 9b. The insertion device 1, 2 is releasably connected to a receiver 11 via hooks 12 on the receiver 11 engaging with recesses 13 formed in the cannula-holding part 5, which receiver 11 is mounted on a base part 10 which base part 10 is fastened to the patient's skin. The base plate 10 is provided with fastening means 14 for fastening of a delivery device (not shown).

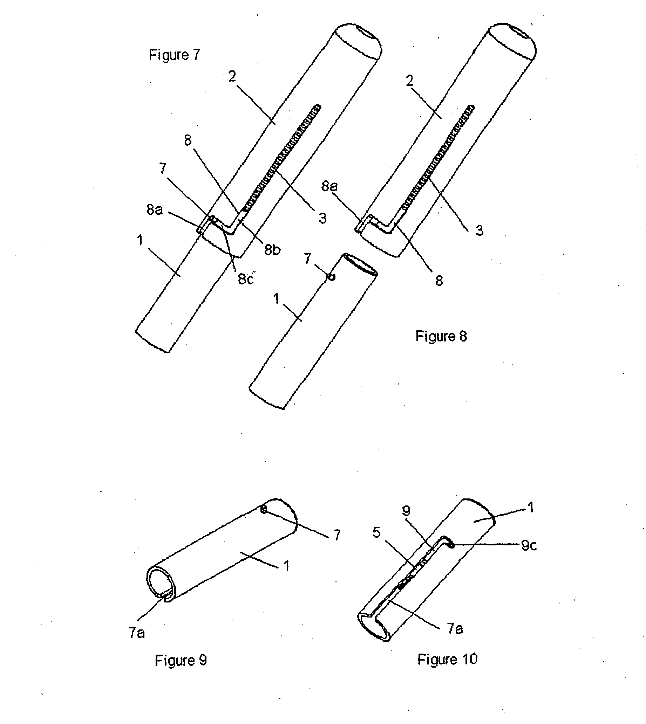

Figure 7 and 8 show in detail the interconnection of the two-unit insertion device1, 2 of the present invention intended for multiple-use. The first insertion part 1 is on the exterior surface provided with a protrusion formed as a cylindrical tap 7 positioned at a surface facing towards the second insertion part 2. The tap 7 interacts with an opening in the form of a slit 8 in the second insertion part 2, said slit 8 is shaped essentially as a stair with a long longitudinal part 8b, a short longitudinal part 8a and a transverse short part 8c separating the two longitudinal parts 8a and 8b, the two longitudinal parts 8a, 8b being parallel and displaced from each other. The transverse short part 8c can be provided with a not shown resting position for the tap 7 e.g. in the form of a depression in the proximal edge of the slit 8c which depression should be wide enough for the tap 7 to fit into in order to make it necessary to press the two insertion parts together before it will be possible to move the tap either to the left or to the right. The short longitudinal part 8a of the slit 8 forms an opening in the proximal edge of the second insertion part 2; the short longitudinal part 8a thereby provides means for uniting or releasing the first insertion part 1 from the second insertion part 2. This embodiment makes it possible to make the two units of the two-unit insertion device 1, 2 interact thereby constituting a needle protector both before and after activation of the insertion device 1, 2 for insertion of the cannula-holding device 5.

When the insertion device 1, 2 is assembled, the tap 7 of the first insertion part 1 is placed in the longitudinal slit 8a of the second insertion part 2 and the first and the second insertion parts are pushed together until the tap 7 reaches corner between the longitudinal slit 8a and the short transverse part 8c of the slit 8. Then the second insertion part 2 is rotated to the left placing the tap 7 in the short transverse part 8c of the slit 8 thereby placing the insertion device in a locked position. The elastic element 3 is in this position unbiased or only slightly biased. The insertion device 1, 2 is now ready for use. Likewise, the insertion device can be disassembled by reverse rotation of the second insertion part 2, thereby moving the tap 7 from the short transverse part 8c of the slit 8 into the short longitudinal slit 8a and drawing the second insertion part 2 away from the first insertion part 1 causing the tap 7 to exit via the short longitudinal slit 8a and thereby releasing the first insertion part 1 from the second insertion part 2.

This embodiment makes it possible to remove and dispose of only the first insertion part 1 and the insertion needle 6 of the two-unit insertion device 1,2 thereby providing a possibility of repeated use of the second insertion 2 together with a new replaced first insertion part 1 containing a new insertion needle 6. Furthermore, this embodiment makes it possible for the first insertion part 1 to constitute a needle protector both before and after activation of the insertion device 1, 2 for insertion of the cannula-holding device 5.

As shown in detail in figure 9 and 10, the first insertion part 1 of the present multi-use embodiment comprises the tap 7 for engaging with the second insertion part 2 and means in the form of a protruding tap 9c which engages with an essentially L-shaped slit 7a for locking and unlocking the needle-holding part 9 during insertion. The needle-holding part 9 comprises the insertion needle 6 (not shown), which needle-holding part 9 is fastened to the cannula-holding part 5.

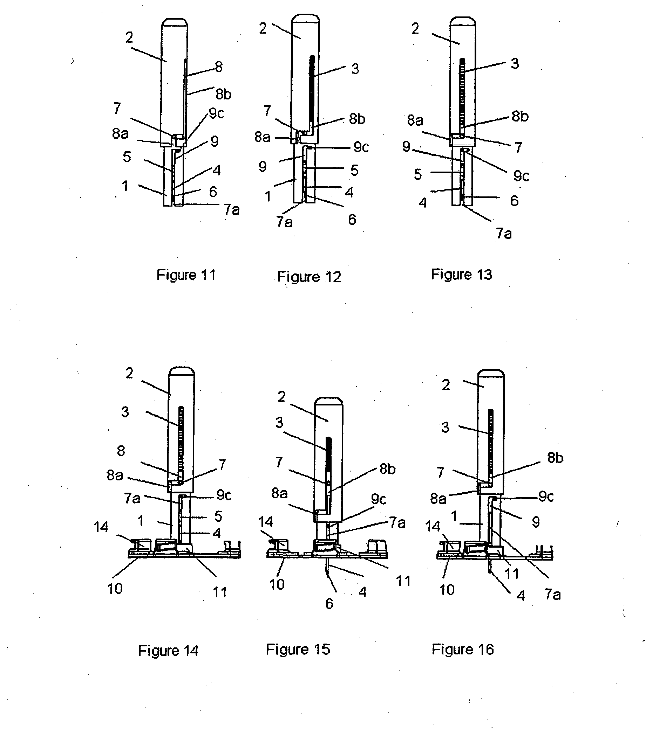

Figures 11-17 show the insertion device 1, 2 when in use. Figure 11 shows the placing and assembling of a disposable first insertion part 1 into a reusable second insertion part 2. The tap 7 of the first insertion part 1 is placed into the short longitudinal slit 8a and moved up to the transverse short part 8c, which part 8c separates the short longitudinal slit 8a from the long longitudinal slit 8b in the stair-shaped slit 8. The disposable first insertion part 1 comprises a needle-holding part 9 with an insertion needle 6 fastened to a cannula-holding part 5. The needle holding part 9 is fixed in relation to the second insertion part 2 and a tap 9c protruding from the exterior surface 1 of the needle-holding part 9 is engaged with slit 7a of the first insertion part 1, the protruding tap 9c is placed in the short part of the L-shaped slit 7a for securing a locking position of the needle-holding part 9 relative to the first insertion part 1. This way, the insertion needle 6 is locked inside in the first insertion part 1 and kept safe and hidden to the patient.

In figure 12, the first insertion part 1 of the insertion device 1, 2 is rotated towards the right thereby moving the tap 7 into the transverse slit 8c separating the short longitudinal slit 8a from the long longitudinal slit 8b thereby securing the first insertion part 1 in a locked position in the longitudinal direction relative to the second insertion part 2. The tap 9c of the needle-holding part 9 is due to the rotation of the first insertion part 1 simultaneously being moved to the left into the short part of the L-shaped slit 7a but the tap 9c remains in a position where the needle-holding part 9 is locked in the longitudinal direction. The elastic element 3 for activating the insertion device 1, 2 is in this position unbiased or slightly biased.

Figure 13 shows the placing of tap 7 at the end point of rotation of the first insertion part 1. The tap 7 has reached the corner between the long longitudinal slit 8b and the transverse slit 8c and further rotation of the first insertion part 1 is not possible. The tap 9c of the needle-holding part 9 is during the rotation of the first insertion part 1 simultaneously moved into the corner of the essentially L-shaped slit 7a leaving the needle-holding part 9 in an unlocked position. The elastic element 3 for activating the insertion device 1, 2 is still in an unbiased position. Thus, the insertion device 1, 2 is left in a position ready for activation and insertion of the cannula 4.

Figure 14 shows the same insertion device 1, 2 as figure 13 in a position ready for activation and insertion of the cannula-holding part 5, where the insertion device 1, 2 is mounted in a receiver 11 connected to a base plate 10. The tap 7 is in an unlocked position in the corner between the long part 8b of the slit 8 and the short transverse part 8c.

Figure 15 shows the activation of the insertion device 1, 2. The second insertion part 2 is manually pressed down towards the patients skin, thereby biasing the elastic element 3 and the first insertion part 1 will slide into the second insertion part 2. At the same time the manually pressure will cause tap 7 to slide in the long longitudinal slit 8b of the second insertion part 2 and tap 9c to slide in the longitudinal slit 7a of the first insertion part 1 by which the insertion needle 6 and cannula 4 exits the first insertion part 1, the insertion needle 6 penetrating of the patients skin and inserting the cannula 4 into the patient. The cannula-holding part 5 engages with the receiver 11 on the base plate 10 which assures fastening of the cannula-holding part 5 to the receiver 11.

Figure 16 shows the insertion device 1, 2 mounted in a receiver 11, which receiver 11 is connected to a base plate 10. The insertion device 1, 2 is shown in a position after insertion of the cannula 4 into a patient. The manual pressure on the second insertion part 2 is released causing the elastic element 3 to move into an unbiased position, this causing the tap 7 to slide down in the long longitudinal slit 8b to the transverse slit separating the short longitudinal slit 8a from the long longitudinal slit 8b, and the tap 9c of the first insertion part 1 to slide up in the longitudinal slit 7a. Thus, the elastic element 3 retracts the insertion needle 6 fastened to the needle-holding part 9 from the cannula-holding part 5 and into the first insertion part 1 leaving the cannula 4 within the patient. The second insertion part 2 is rotated to the left causing the tap 7 to move into the transverse part separating the two long and short longitudinal slits 8a,8b, and causing tap 9c to move into the short part of the essentially L-shaped slit 7a, thereby locking both the first insertion part to the second insertion part and locking the needle-holding part 9 within the first insertion part 1. The insertion device 1, 2 is then to be safely removed from the receiver 11, the insertion needle 6 being kept safely inside the first insertion part 1 and not visible to the patient.

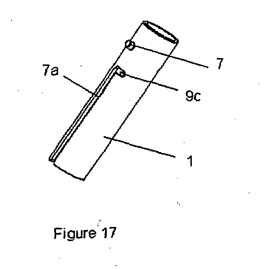

Figure 17 shows the first insertion part 1 in a position after use, where the first insertion part 1 containing the used insertion needle 6 (not shown) is released from the second insertion part 2 for disposal. The needle-holding part 9 (not shown) is locked to the first insertion part 1 via the tap 9c in the essentially L-shaped slit 7a, thereby keeping the used insertion needle 6 (not shown) within the first insertion part 1 for safety reasons, when disposed of.

With the present multi-use embodiment, a used insertion needle can be both safely removed and disposed of after application. The used first insertion part can be replaced with a new first insertion part comprising a new needle and needle-holding part as well as a new cannula-holding part with a new cannula and assembled with the used second insertion part 2. Thus, it is possible to reuse the second insertion part and only replace the first insertion part thereby saving expenses.

## Claims

1. A device for insertion of a cannula into a subcutaneous layer of skin of a patient, said device comprising a first insertion part (1) and a second insertion part (2), a cannula holding part (5) provided with a cannula (4), and an injection needle (6) where
- the second insertion part (2) is connected to the injection needle (6) and the injection needle (6) is releasably combined with the cannula (4) of the cannula holding part (5),
- the first insertion part (1) covers the injection needle (6) in a non-activated position and in an activated position the injection needle (6) projects beyond the first insertion part (1),
- at least a part of the second insertion part (2) and the first insertion part (1) can be moved in relation to each other between at least one activated position and at least one non-activated position,
and the second insertion part (2) exceeds the distal end of the first insertion part (1) when the device is in an activated position
**characterized in that** an elastic element (3) supports bringing the second insertion part (2) which is connected to the injection needle (6) from a forward i.e. an activated position to a retracted i.e. a non-activated position.

2. A device for insertion of a cannula according to claim 1 **characterized in that** the elastic element (3) comprises a spring being in contact respectively with a surface of the second insertion part (2) and a surface of the first insertion part (1).

3. A device for insertion of a cannula according to any of the claims 1-2 **characterized in that** a part (2b) of the second insertion part (2) moves between at least one forward position and at least one retracted position surrounding the first insertion part (1).

4. A device for insertion of a cannula according to any of the claims 1-3 **characterized in that** the second insertion part (2) and the first insertion part (1) are provided with interacting guiding means (7, 8) for guiding the first and second insertion parts (1. 2) in relation to each other in a slidable movement over a certain distance.

5. A device for insertion of a cannula according to claim 1, 2 3 or 4, wherein the first and the second insertion parts (1, 2) are releasably connected to each other.

6. A device for insertion of a cannula according to any of the claims 1-5 wherein the first insertion part (1) comprises means for releasably connecting the first insertion part to a corresponding receiving portion (11) placed on a patient's skin.

7. A device for insertion of a cannula according to claim 6 wherein the proximal end of the first insertion part (1) is shaped as a cylindrical tube part releasably connected to a corresponding cylindrical portion (11) placed on a surface on the skin of a patient.

8. A device for insertion of a cannula according to claims 6 or 7 wherein the receiving portion (11) is attached to a base part (10) which is positioned on a patient's skin before injection of the cannula (4) of the cannula-holding part (5).

9. A device for insertion, of a cannula according to any of the preceding claims, where the elastic element (3) is unbiased when the first and the second insertion parts (1, 2) are in a non-activated position.

10. A device for insertion of a cannula according to any of the claims 1-9, where the first insertion part (1) is supported against a surface (10, 11) while the guiding means (7, 8) lead the injection needle (6) which is connected to the second insertion part (2) through the surface and back again.

11. A device for insertion of a cannula according to any of the claims 1-10, where the first insertion part (1) is formed as a pipe section while the second insertion part (2) is at least partly formed as a plunger corresponding to the interior of the first insertion part (1).

12. A device for insertion of a cannula according to any of the claims 1-11, where injection of the injection needle (6) is done by manually pushing the second insertion part (2) towards the surface of injection.

13. A device for insertion of a cannula according to claim 5, where the needle holding part (9) of the second insertion part (2) including the injection needle (6) is retained in the first insertion part (1) when the first and the second insertion parts (1, 2) are released from each other.

14. A device for insertion of a cannula according to any of the claims 1-13, comprising locking means (7, 8) which locking means can lock the position of the first insertion part (1) in relation to the second insertion part (2) in the non-activated position.

15. A device for insertion of a cannula according to claim 14, which locking means (7, 8) comprises a protruding part (7) positioned on the outer surface of the first insertion part (1) combined with an opening or slit (8) in the surface of the second insertion part (2).

## Patentansprüche

1. Vorrichtung zum Einführen einer Kanüle in eine subkutane Hautschicht eines Patienten, welche Vorrichtung ein erstes Einführteil (1) und ein zweites Einführteil (2), ein mit einer Kanüle (4) ausgestattetes Kanülehalteteil (5) und eine Injektionsnadel (6) umfasst, wobei
- das zweite Einführteil (2) mit der Injektionsnadel (6) verbunden ist, und die Injektionsnadel (6) mit der Kanüle (4) des Kanülehalteteils (5) lösbar kombiniert ist,
- das erste Einführteil (1) die Injektionsnadel (6) in einer nichtbetätigten Position abdeckt, und die Injektionsnadel (6) in einer betätigten Position über das erste Einführteil (1) hinaus vorspringt,
- zumindest ein Teil des zweiten Einführteils (2) und das erste Einführteil (1) zwischen zumindest einer betätigten Position und zumindest einer nichtbetätigten Position relativ zueinander bewegt werden können,
und das zweite Einführteil (2) über das distale Ende des ersten Einführteils (1) hinausgeht, wenn sich die Vorrichtung in einer betätigten Position befindet,
**dadurch gekennzeichnet, dass** ein elastisches Element (3) das Bringen des mit der Injektionsnadel (6) verbundenen zweiten Einführteils (2) aus einer vorderen Position, d.h. einer betätigten Position, in eine zurückgezogene, d.h. eine nichtbetätigte Position, unterstützt.

2. Vorrichtung zum Einführen einer Kanüle nach Anspruch 1, **dadurch gekennzeichnet, dass** das elastische Element (3) eine Feder umfasst, welche sich mit einer Fläche des zweiten Einführteils (2) bzw. einer Fläche des ersten Einführteils (1) in Kontakt befindet.

3. Vorrichtung zum Einführen einer Kanüle nach einem der Ansprüche 1 bis 2, **dadurch gekennzeichnet, dass** sich ein Teil (2b) des zweiten Einführteils (2) zwischen zumindest einer vorderen Position und zumindest einer zurückgezogenen Position bewegt, indem es das erste Einführteil (1) umgibt.

4. Vorrichtung zum Einführen einer Kanüle nach einem der Ansprüche 1 bis 3, **dadurch gekennzeichnet, dass** das zweite Einführteil (2) und das erste Einführteil (1) mit zusammenwirkenden Führungsmitteln (7, 8) zum Führen des ersten und des zweiten Einführteils (1, 2) relativ zueinander in einer Gleitbewegung über eine bestimmte Strecke ausgestattet sind.

5. Vorrichtung zum Einführen einer Kanüle nach Anspruch 1, 2, 3 oder 4, wobei das erste und das zweite Einführteil (1, 2) miteinander lösbar verbunden sind.

6. Vorrichtung zum Einführen einer Kanüle nach einem der Ansprüche 1 bis 5, wobei das erste Einführteil (1) Mittel zum lösbaren Verbinden des ersten Einführteils mit einem entsprechenden Aufnahmeteil (11) umfasst, welches auf der Haut eines Patienten angeordnet ist.

7. Vorrichtung zum Einführen einer Kanüle nach Anspruch 6, wobei das proximale Ende des ersten Einführteils (1) als ein zylindrisches Rohrteil ausgebildet ist, welches mit einem entsprechenden zylindrischen Abschnitt (11) lösbar verbunden ist, welcher auf einer Oberfläche auf der Haut eines Patienten angeordnet ist.

8. Vorrichtung zum Einführen einer Kanüle nach Anspruch 6 oder 7, wobei das Aufnahmeteil (11) an einem Basisteil (10) befestigt ist, welches vor Injektion der Kanüle (4) des Kanülehalteteils (5) auf der Haut eines Patienten angeordnet ist.

9. Vorrichtung zum Einführen einer Kanüle nach einem der vorgehenden Ansprüche, wobei das elastische Element (3) entspannt ist, wenn sich das erste und das zweite Einführteil (1, 2) in einer nichtbetätigten Position befinden.

10. Vorrichtung zum Einführen einer Kanüle nach einem der Ansprüche 1 bis 9, wobei sich das erste Einführteil (1) gegen eine Fläche (10, 11) stützt, während die Führungsmittel (7, 8) die mit dem zweiten Einführteil (2) verbundene Injektionsnadel (6) durch die Fläche hindurch und wieder zurück führen.

11. Vorrichtung zum Einführen einer Kanüle nach einem der Ansprüche 1 bis 10, wobei das erste Einführteil (1) als ein Rohrabschnitt ausgebildet ist, während das zweite Einführteil (2) als ein dem Inneren des ersten Einführteils (1) entsprechender Kolben zumindest teilweise ausgebildet ist.

12. Vorrichtung zum Einführen einer Kanüle nach einem der Ansprüche 1 bis 11, wobei die Injektion der Injektionsnadel (6) durch ein manuelles Drücken des zweiten Einführteils (2) gegen die Injektionsfläche durchgeführt wird.

13. Vorrichtung zum Einführen einer Kanüle nach Anspruch 5, wobei das Nadelhalteteil (9) des zweiten Einführteils (2) umfassend die Injektionsnadel (6) in dem ersten Einführteil (1) festgehalten ist, wenn das erste und das zweite Einführteil (1, 2) voneinander gelöst werden.

14. Vorrichtung zum Einführen einer Kanüle nach einem der Ansprüche 1 bis 13, umfassend Verschlussmittel (7, 8), welche Verschlussmittel die Position des ersten Einführteils (1) relativ zum zweiten Einführteil (2) in der nichtbetätigten Position verschließen können.

15. Vorrichtung zum Einführen einer Kanüle nach Anspruch 14, welche Verschlussmittel (7, 8) ein an der Außenfläche des ersten Einführteils (1) angeordnetes vorspringendes Teil (7) umfassen, welches mit einer Öffnung oder einem Schlitz (8) in der Oberfläche des zweiten Einführteils (2) kombiniert ist.

## Revendications

1. Dispositif pour l'insertion d'une canule dans une couche sous-cutanée de la peau d'un patient, ledit dispositif comprenant une première pièce d'insertion (1) et une deuxième pièce d'insertion (2), une partie de retenue de la canule (5) pourvue d'une canule (4), et une aiguille d'injection (6), où
- la deuxième pièce d'insertion (2) est reliée à l'aiguille d'injection (6) et l'aiguille d'injection (6) est combinée de manière amovible avec la canule (4) de la partie de retenue de la canule (5),
- la première pièce d'insertion (1) couvre l'aiguille d'injection (6) dans une position non activée, et dans une position activée l'aiguille d'injection (6) fait saillie au-delà de la première pièce d'insertion (1),
- au moins une portion de la deuxième pièce d'insertion (2) et la première pièce d'insertion (1) peuvent être déplacées l'une par rapport de l'autre entre au moins une position activée et au moins une position non activée,
et le deuxième pièce d'insertion (2) dépasse l'extrémité distale de la première pièce d'insertion (1) lorsque le dispositif est dans une position activée, **caractérisé en ce qu'**un élément élastique (3) aide à amener la deuxième pièce d'insertion (2) qui est reliée à l'aiguille d'injection (6) à partir d'une position en avant, c'est-à-dire activée, à une position rétractée, c'est-à-dire non activée.

2. Dispositif pour l'insertion d'une canule selon la revendication 1, **caractérisé en ce que** l'élément élastique (3) comprend un ressort étant en contact respectivement avec une surface de la deuxième pièce d'insertion (2) et une surface de la première pièce d'insertion (1).

3. Dispositif pour l'insertion d'une canule selon l'une quelconque des revendications 1 à 2, **caractérisé en ce qu'**une portion (2b) de la deuxième pièce d'insertion (2) se déplace entre au moins une position avant et au moins une position rétractée entourant la première pièce d'insertion (1).

4. Dispositif pour l'insertion d'une canule selon l'une quelconque des revendications 1 à 3, **caractérisé en ce que** la deuxième pièce d'insertion (2) et la première pièce d'insertion (1) sont pourvues de moyens de guidage (7, 8) l'un agissant avec l'autre pour guider les première et deuxième pièces d'insertion (1, 2) l'une par rapport à l'autre dans un mouvement glissant sur une certaine distance.

5. Dispositif pour l'insertion d'une canule selon la revendication 1, 2, 3 ou 4, dans lequel les première et deuxième pièces d'insertion (1, 2) sont reliées l'une à l'autre de manière amovible.

6. Dispositif pour l'insertion d'une canule selon l'une quelconque des revendications 1 à 5, dans lequel la première pièce d'insertion (1) comprend des moyens pour relier de manière amovible la première pièce d'insertion à une partie correspondante de réception (11) placée sur la peau d'un patient.

7. Dispositif pour l'insertion d'une canule selon la revendication 6, dans lequel l'extrémité proximale de la première pièce d'insertion (1) présente la forme d'une partie de tube cylindrique reliée de façon amovible à une partie cylindrique correspondante (11) placée sur une surface sur la peau d'un patient.

8. Dispositif pour l'insertion d'une canule selon la revendication 6 ou 7, dans lequel la partie de réception (11) est attachée à une partie de base (10) qui est positionnée sur la peau d'un patient avant l'injection de la canule (4) de la partie de retenue de la canule (5).

9. Dispositif pour l'insertion d'une canule selon l'une quelconque des revendications précédentes, dans lequel l'élément élastique (3) est non-biaisé lorsque les première et deuxième pièces d'insertion (1, 2) sont dans une position non activée.

10. Dispositif pour l'insertion d'une canule selon l'une quelconque des revendications 1 à 9, dans lequel la première pièce d'insertion (1) est en appui contre une surface (10, 11) tandis que les moyens de guidage (7, 8) guident l'aiguille d'injection (6) qui est reliée à la deuxième pièce d'insertion (2) à travers la surface et de retour.

11. Dispositif pour l'insertion d'une canule selon l'une quelconque des revendications 1 à 10, dans lequel la première pièce d'insertion (1) est formée comme une section de tuyau alors que la deuxième pièce d'insertion (2) est au moins partiellement réalisée sous la forme d'un piston correspondant à l'intérieur de la première pièce d'insertion (1).

12. Dispositif pour l'insertion d'une canule selon l'une quelconque des revendications 1 à 11, dans lequel l'injection de l'aiguille d'injection (6) se fait manuellement en poussant la deuxième pièce d'insertion (2) en direction de la surface d'injection.

13. Dispositif pour l'insertion d'une canule selon la revendication 5, dans lequel la partie de retenue de l'aiguille (9) de la deuxième pièce d'insertion (2) comportant l'aiguille d'injection (6) est retenue dans la première pièce d'insertion (1) lorsque les première et deuxième pièces d'insertion (1, 2) sont libérées l'une de l'autre.

14. Dispositif pour l'insertion d'une canule selon l'une quelconque des revendications 1 à 13, comprenant des moyens de verrouillage (7, 8), lesdits moyens de verrouillage pouvant verrouiller la position de la première pièce d'insertion (1) par rapport à la deuxième pièce d'insertion (2) dans la position non activée.

15. Dispositif pour l'insertion d'une canule selon la revendication 14, lesdits moyens de verrouillage (7, 8) comprennent une partie en saillie (7) positionnée sur la surface extérieure de la première pièce d'insertion (1) en combinaison avec une ouverture ou une fente (8) dans la surface de la deuxième pièce d'insertion (2).
